(19) Europäisches Patentamt
European Patent Office
Office européen des brevets

(11) **EP 2 578 265 B1**

(12) **EUROPEAN PATENT SPECIFICATION**

(45) Date of publication and mention
of the grant of the patent:
**03.04.2019 Bulletin 2019/14**

(51) Int Cl.:
***A61M 37/00*** *(2006.01)*

(21) Application number: **11786694.7**

(22) Date of filing: **25.05.2011**

(86) International application number:
**PCT/JP2011/062026**

(87) International publication number:
**WO 2011/148995 (01.12.2011 Gazette 2011/48)**

(54) **ARRAY WITH FINE PROTRUSIONS**

ARRAY MIT FEINEN ERHEBUNGEN

RÉSEAU À FINES PROTUBÉRANCES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2010 JP 2010122989**

(43) Date of publication of application:
**10.04.2013 Bulletin 2013/15**

(73) Proprietors:
• **Hisamitsu Pharmaceutical Co., Inc.**
**Tosu-shi, Saga 841-0017 (JP)**
• **Toppan Printing Co., Ltd.**
**Tokyo 110-0016 (JP)**

(72) Inventors:
• **TOKUMOTO Seiji**
**Tsukuba-shi**
**Ibaraki 305-0856 (JP)**
• **ISHITSUKA Shuji**
**Tsukuba-shi**
**Ibaraki 305-0856 (JP)**
• **KODAMA Yoshihiro**
**Tokyo 110-0016 (JP)**
• **TAKEMURA Nobumi**
**Tokyo 110-0016 (JP)**

(74) Representative: **Grünecker Patent- und Rechtsanwälte**
**PartG mbB**
**Leopoldstraße 4**
**80802 München (DE)**

(56) References cited:
**EP-A1- 2 383 013        WO-A1-2004/009172**
**JP-A- 2006 149 818     JP-A- 2006 149 818**
**JP-A- 2007 089 792     JP-A- 2007 130 417**
**JP-A- 2007 130 417     JP-A- 2008 509 723**
**JP-A- 2008 522 731**

**Description**

**Technical Field**

The present invention relates to an array with microprotrusions for administering an active ingredient via the skin.

**Background Art**

Heretofore, one in which microprotrusions are disposed on a base (array with microprotrusions) has been known as an array for improving the transdermal absorption of drugs. The microprotrusions are aimed at puncturing the stratum corneum, which is the outermost layer of the skin, and various sizes or shapes have been proposed (see Patent Literature 1).
Moreover, various methods have also been proposed as to methods for applying drugs in the case of using the array with microprotrusions. In Patent Literature 2, it is described that: a drug is coated onto the surfaces of the microprotrusions; grooves or hollow portions for permeating a drug or a biogenic substance are disposed in the microprotrusions; a drug is mixed with the microprotrusions themselves; etc. Moreover, in Patent Literature 2, it is preferred that a reservoir medium should contain sugars, and it is also described that the reservoir medium particularly contains sugars for stabilization that forms glass (amorphous solid substance), such as lactose, raffinose, trehalose, or sucrose.
Furthermore, in Patent Literatures 3 and 4, there is the description that by setting the height of the micropro-trusions to 10 μm to 3 mm and setting the shape of the tips of the microprotrusions to a flat shape or a rounded shape, the microprotrusions can administer cosmetics, pharmaceuticals, or compounds such as plastics attached to or contained in the protrusions while stretching the epidermis without penetrating the stratum corneum.

**Citation List**

**Patent Literature**

    Patent Literature 1: JP 2001-506904
    Patent Literature 2: JP 2004-504120
    Patent Literature 3: JP 2007-089792
    Patent Literature 4: JP 2007-130417

**Summary of Invention**

**Technical Problem**

In the case of administering an active ingredient to a sensitive portion in the skin, it is preferred not to completely penetrate the stratum corneum, in order to avoid the possibility that the skin gets damaged. The reason therefor is that when a through-hole is formed in the stratum corneum, the occurrence of skin irritation (erythema) or reduction in the water-holding capacity of the skin resulting from increase in water loss from the skin is caused. However, the patterns of shapes or heights of protrusions in the transdermal administration apparatus described in Patent Literatures 3 and 4 above are enormous, and it is not said to disclose the optimum array.
Thus, an array with microprotrusions has been demanded, which is capable of administering an active ingredient to the skin without pain and with reliability while preventing damage to the stratum corneum of the skin.

**Solution to Problem**

During the course of diligent study to solve the problems described above, the present inventors have found that merely setting the shape and density of microprotrusions may result in perforation of the corneum, and it is important to consider the extent to which the skin stretches.
Specifically, an array with microprotrusions according to an embodiment of the present invention comprises: a base; and tapered microprotrusions each disposed on the base and tapering down toward the tip from the bottom connected to the base, wherein letting a distance from the tip to the bottom on an arbitrary side of each of the microprotrusions as a and letting the length of a second line segment prepared by projecting a first line segment representing the distance onto the base as b, a relationship of $1.0 < (a/b) \le 7.5$ holds.
The invention is defined by the appended claims. Subject matter referred as embodiments and/or inventions which are not claimed are not part of the invention.

According to such an array with microprotrusions, the skin at a site coming into contact with each protrusion when the microprotrusion is placed thereagainst stretches by a value of a/b described above at the maximum. In this context, the value a/b represents the rate of stretching provided that the skin has been stretched completely along the side of the microprotrusion, and can be said to be the maximum rate of stretching of the skin by the microprotrusion. The present inventors have found that if this maximum rate of stretching can be kept at 7.5 or less, there is no risk of penetrating the stratum corneum of the skin. Specifically, by determining the shape of the microprotrusion so as to satisfy a relationship of $1.0 < (a/b) \leq 7.5$, an active ingredient can be administered to the skin without pain and with reliability via the stratum corneum that has thinned out by stretching, with damage to the stratum corneum prevented.

An array with microprotrusions according to another example comprises: a base; and microprotrusions, wherein the microprotrusions stretch the skin by 1.01 to 3.0 times. According to such an array with microprotrusions, since the rate of stretching of the skin can be kept at 1.01 to 3.0, an active ingredient can be administered to the skin without pain and with reliability via the stratum corneum that has thinned out by stretching, with damage to the stratum corneum prevented.

In the array with microprotrusions according to the invention, the microprotrusions may be made of polylactic acid. Since the polylactic acid is biodegradable, in this case, burdens on the skin, etc., can be reduced even if the microprotrusions remain on the skin by breaking or the like. Moreover, the polylactic acid is also advantageous in terms of antigenicity and the unit price of a material.

In the array with microprotrusions according to a further alternative example, the height of the microprotrusions may be 20 to 400 $\mu$m, and the width of the bottom may be 10 to 200 $\mu$m.

In the array with microprotrusions according to the invention, the density of the microprotrusions may be 100 to 10000 protrusions/cm$^2$.

In the array with microprotrusions according to the invention, the tip is flat, and the area of the tip is 20 to 600 $\mu$m$^2$. In this case, since pressure against the skin contacted with the tip of the microprotrusion is reduced, damage to this site can be avoided more reliably.

In the array with microprotrusions according to a further alternative example, the tip may be rounded, and the radius of curvature of the tip may be 2 to 100 $\mu$m. In this case, since pressure against the skin contacted with the tip of the microprotrusion is reduced, damage to this site can be avoided more reliably.

In the array with microprotrusions according to a further alternative example, the tip may be pointed, and the apical angle of the tip projected onto an arbitrary reference surface orthogonal to the base may be 16 degrees or larger. Since the value a/b can thereby be within the range of $1.0 < (a/b) \leq 7.5$, an active ingredient can be administered to the skin without pain and with reliability, with damage to the stratum corneum prevented even if the microprotrusions are pointed.

In the array with microprotrusions according to a further alternative example, the microprotrusions may not penetrate the stratum corneum of the skin.

## Advantageous Effects of Invention

According to an aspect of the present invention, letting a distance from the tip to the bottom on a side of each microprotrusion as a and letting the length of a second line segment prepared by projecting a first line segment representing the distance onto the base as b, a relationship of $1.0 < (a/b) \leq 7.5$ holds. By thus determining the shape of the microprotrusion, an active ingredient can be administered to the skin without pain and with reliability, with damage to the stratum corneum of the skin prevented.

## Brief Description of Drawings

Figure 1 is a perspective view showing one example of an array with microprotrusions according to an embodiment.
Figure 2 is a sectional view taken along the II-II line in Figure 1.
Figure 3(a) is a perspective view of a conical microprotrusion; Figure 3(b) is a sectional view taken along the B-B line in Figure 3(a); Figure 3(c) is a perspective view of a quadrangular pyramidal microprotrusion; and Figure 3(d) is a sectional view taken along the D-D line in Figure 3(c).
Figure 4 is a graph showing water loss in Example 3.
Figure 5 is a graph showing change in impedance in Example 4.
Figure 6 is a diagram schematically showing the state in which the skin has been stretched incompletely along the microprotrusion.

**Description of Embodiments**

Hereinafter, embodiments of the present invention will be described in detail with reference to the accompanying drawings. Incidentally, in the description of the drawings, the same reference signs will be used to designate the same or similar components, so that the description will be omitted.

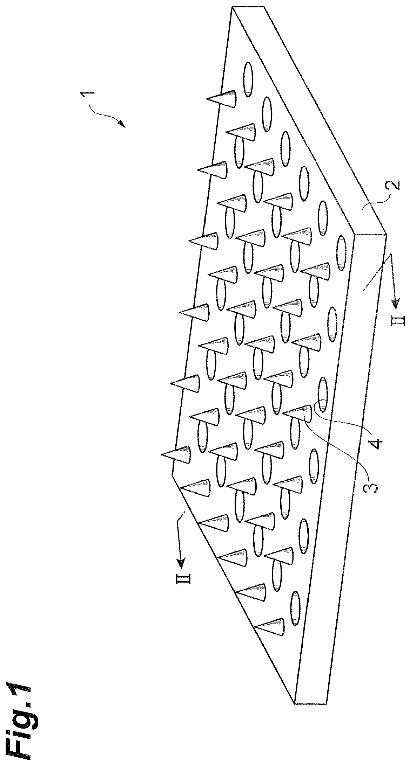

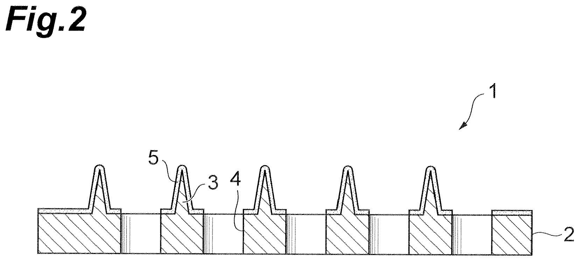

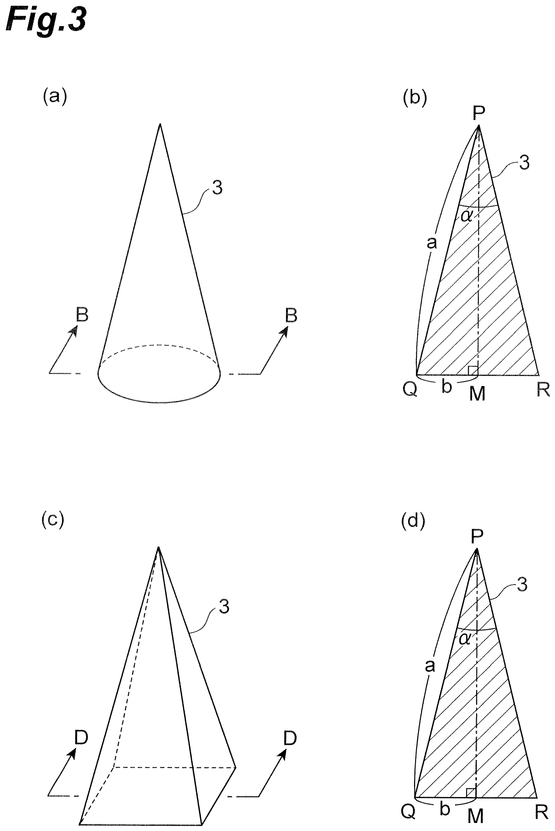

First, the constitution of an array 1 with microprotrusions (hereinafter, also simply referred to as an "array 1") according to an embodiment will be described. In the present specification, the "array" is merely the name of an instrument. Figure 1 is a perspective view showing one example of the array 1 according to an embodiment. Figure 2 is a sectional view taken along the II-II line in Figure 1. Figure 3 is a perspective view and a sectional view of a microprotrusion 3.

As shown in Figure 1, the array 1 comprises a base 2 and a plurality of microprotrusions 3 arranged in a two-dimensional pattern on the base 2.

The base 2 is a foundation for supporting the microprotrusions 3. In the base 2, a plurality of through-holes 4 are formed so as to be arranged in a two-dimensional pattern. The microprotrusions 3 and the through-holes 4 are alternately arranged in the diagonal direction of the base 2. By means of the through-holes 4, it becomes possible to administer a biologically active ingredient from the back of the base 2. As a matter of course, a base free from such through-holes may be used. The area of the base 2 may be 0.5 cm² to 300 cm², may be 1 cm² to 100 cm², or may be 1 cm² to 50 cm². A base of the desired size may be constituted by connecting several individuals of this base 2.

Each microprotrusion 3 is a microstructure, and its height (length) h is, for example, 20 to 400 $\mu$m. In this context, the reason why the length of the microprotrusion 3 is set to 20 $\mu$m or larger is that the transdermal administration of an active ingredient is secured, and the reason for setting to 400 $\mu$m or smaller is that the microprotrusion is prevented more reliably from piercing the corneum of the skin. Alternatively, if the length of the microprotrusion 3 is 300 $\mu$m or smaller, an active ingredient in an amount that should enter intradermally can be administered efficiently. The length of the microprotrusion 3 may be 50 to 300 $\mu$m.

In this context, the microprotrusion is a tapered structure tapering down toward the tip from the bottom connected to the base 2 and means a needle shape in a broad sense or a structure containing a needle shape. As a matter of course, the microprotrusion is not limited to the needle shape having a sharp tip and also includes a shape free from a pointed end. In the case where the microprotrusion 3 has a conical structure, the diameter at its base may be on the order of 5 to 250 $\mu$m or may be 10 to 200 $\mu$m. Although conical microprotrusions 3 are shown in Figure 1, microprotrusions in a polygonal pyramidal shape such as a quadrangular pyramidal shape may be used.

Letting a distance from the tip to the bottom on a side (along a side) of the microprotrusion 3 as a and letting the length of a line segment prepared by projecting a line segment representing the distance a onto the base 2 as b, the relationship represented by the following formula (1) may hold:

$$1.0 < (a/b) \leq 7.5 \ ... \ (1)$$

As an example, the case where the microprotrusion 3 is conical/quadrangular pyramidal is shown in Figure 3. In the case where the microprotrusion 3 is conical as shown in Figure 3(a), a line segment representing a distance a from a tip P to the bottom on its side is an oblique side PQ in a triangle PQR (sectional view including the tip P) of Figure 3(b). Moreover, a line segment prepared by projecting the oblique side PQ onto the base 2 along a direction orthogonal to the base 2 is a line segment QM in the triangle PQR. In this context, a point M is the foot of a perpendicular from the point P to the base QR. Even if the microprotrusion 3 is quadrangular pyramidal as shown in Figure 3(c), the same as in the conical case can hold true therefor as shown in Figure 3(d).

In this context, the value a/b is the rate of stretching provided that the skin in a normal state has been stretched completely along the side of the microprotrusion 3, i.e., an index representing by what times the skin is stretched by the microprotrusion 3 at the maximum, and can be said to be the maximum rate of stretching of the skin by the microprotrusion 3. In the examples of Figure 3, the maximum rate of stretching of the skin may satisfy the above formula (1) in the case where the skin at a site along the line segment QM has been stretched along the oblique side PQ by the placement of the array 1 (contact of the array 1) thereagainst. This is because if the rate of stretching of the skin is kept at this level, damage to the stratum corneum can be avoided more reliably when the array 1 is placed thereagainst. The rate of stretching may be 1.01 to 3.0 or may be 1.01 to 2.0.

In the case where the microprotrusion 3 is conical or pyramidal, the apical angle $\alpha$ (see Figures 3(c) and 3(d)) of the tip projected onto an arbitrary reference surface orthogonal to the base 2 may be 16 degrees or larger (less than 180 degrees). Specifically, the minimum value of the apical angle $\alpha$ of the tip projected onto the arbitrary reference surface may be 16 degrees or larger. Since the maximum rate of stretching of the skin can be kept within the range of the above formula (1) by thus adjusting the apical angle, damage attributed to the microprotrusion can be avoided more reliably.

The above formula (1) holds not only for the examples of Figure 3, but also holds even if the microprotrusion is arbitrarily pyramidal or is arbitrarily oblique conical. However, it is required that the formula (1) should hold for an arbitrary side if the microprotrusion is pyramidal and that the formula (1) should hold for an arbitrary generatrix if conical.

The microprotrusion may not be conical and pyramidal, and, for example, the tip may be flat or may be rounded. In the case where the tip is flat, the area of the flat portion may be 20 to 600 $\mu m^2$ or may be 50 to 250 $\mu m^2$. Alternatively, in the case where the tip is rounded, the radius of curvature of the tip may be 2 to 100 $\mu m$ or may be 5 to 30 $\mu m$. Even if the microprotrusion has such a shape, the above formula (1) holds. Since pressure against the skin contacted with the tip of the microprotrusion is reduced by thus processing the tip of the microprotrusion, damage to this site can be avoided more reliably.

Although the microprotrusions 3 do not penetrate the stratum corneum of the skin in usual use, it is also possible that some microprotrusions 3 penetrate the stratum corneum as long as there is no inconvenience such as inflammation from the viewpoint of skin beauty. Specifically, the epidermis thins out by stretching by the microprotrusions 3, and an active ingredient permeates the epidermis that has been rendered permeable, though it is possible that a portion of the active ingredient enters into the skin from the pierced corneum.

Regarding the density of the microprotrusions 3, typically, spacing is given so that a density of approximately 1 to 10 per mm is provided as to the row of needles. In general, adjacent rows are spaced from each other by a distance substantially equal to the space between the needles in each row, and have a density of 100 to 10000 needles per $cm^2$. The density of the microprotrusions 3 may be 200 to 5000 protrusions or may be 300 to 2000 protrusions or 400 to 850 protrusions.

Examples of the material of the base 2 or the microprotrusions 3 include silicon, silicon dioxide, ceramics, metals (stainless, titanium, nickel, molybdenum, chromium, cobalt, etc.), and synthetic or natural resin materials, but include biodegradable polymers such as polylactic acid, polyglycolide, polylactic acid-co-polyglycolide, pullulan, caprolactone, polyurethane, and polyanhydrides, and synthetic or natural resin materials such as polycarbonate, polymethacrylic acid, ethylene vinyl acetate, polytetrafluoroethylene, and polyoxymethylene, which are non-degradable polymers, in consideration of the antigenicity of the microprotrusions and the unit price of the material. Moreover, hyaluronic acid, sodium hyaluronate, pullulan, dextran, dextrin or chondroitin sulfate, a cellulose derivative, and the like, which are polysaccharides may be used.

The material of the microprotrusions 3 may be a biodegradable resin such as polylactic acid in consideration of breaking on the skin. Although a polylactic acid homopolymer of poly-L-lactic acid or poly-D-lactic acid, a poly-L/D-lactic acid copolymer, and a mixture thereof, etc. are included in the polylactic acid, any of these may be used. Moreover, the larger the average molecular weight of the polylactic acid becomes, the larger its strength becomes, and those of 40,000 to 100,000 can be used.

Examples of the method for producing the base 2 or the microprotrusions 3 include wet etching or dry etching using a silicon base, precision machining using metal or resin (electro-discharge machining, laser processing, grinding, hot embossing, injection molding, etc.), and machinery cutting. By these processing methods, the protrusions and the supporting portion are integrally molded. Examples of the method for rendering the protrusions hollow include a method of performing secondary processing by laser processing or the like after preparation of the protrusions.

Coating 5 with an active ingredient is provided on the base 2 and/or the microprotrusions 3. In the present embodiment, the coating 5 is one in which a coating solution containing a polymer carrier having compatibility with the active ingredient is anchored to a portion or the whole of the microprotrusions 3 and/or surface of the base 2. Examples of the polymer carrier include carboxyvinyl polymers and polyethylene oxide described later, polyvinylpyrrolidone, polyvinyl alcohol, and cellulose derivatives. "Anchored" refers to maintaining the state in which the coating solution is almost evenly attached to an object. Immediately after coating, the coating solution is anchored in a dry state by a known drying method of air drying, vacuum drying, freeze drying, or a combination thereof, but is not limited to anchoring in a dry state because of also retaining a water content in equilibrium with a surrounding atmosphere, an organic solvent, or the like after transdermal administration.

The coating agent contains an active ingredient and purified water and/or a coating carrier. The coating carrier includes polyethylene oxide, hydroxymethyl cellulose, hydroxypropyl cellulose, hydroxypropyl methyl cellulose, methylcellulose, carboxymethyl cellulose, carmellose sodium, dextran, polyethylene glycol, polyvinyl alcohol, polyvinylpyrrolidone, chondroitin sulfate, hyaluronic acid, sodium hyaluronate, dextrin, gum arabic, ethanol, isopropanol, methanol, propanol, butanol, dimethyl sulfoxide, N,N-dimethylformamide, polyethylene glycol, benzyl benzoate, sesame oil, soybean oil, lactic acid, benzyl alcohol, polysorbate 80, alpha thioglycerin, ethylenediamine, N,N-dimethylacetamide, thioglycolic acid, phenoxyethanol, etc.

The content of the coating carrier in the coating agent may be 0.1 to 70% by weight. Moreover, the coating carrier may be viscous to some extent so as not to drip.

A liquid composition used for coating the base 2 and/or the microprotrusions 3 is prepared by mixing a biocompatible carrier, a beneficial active ingredient to be delivered, and, in some cases, any coating auxiliary with a volatile liquid. The volatile liquid can be water, dimethyl sulfoxide, dimethylformamide, ethanol, isopropyl alcohol, and a mixture thereof. For example, water may be selected. The liquid coating solution or suspension can typically have 0.1 to 65% by weight of a beneficial biologically active ingredient concentration, and the concentration may be 1 to 40% by weight or 10 to 30% by weight. The coating may become an anchored state. A surfactant may be zwitterionic, amphoteric, cationic, anionic, or nonionic. For example, Tween 20 and Tween 80, other sorbitan derivatives, for example, sorbitan laurate, and alkoxylated alcohols, for example, laureth-4, may be used. For example, the addition of a surfactant is also effective for dissolving a larger amount of the active ingredient in the coating carrier.

The active ingredient used in the present embodiment is selected without particular limitations from the group consisting of antioxidants, free radical scavengers, humectants, depigmentation agents, fat controllers, UV-reflecting agents, wetting agents, antimicrobial agents, antiallergic drugs, anti-acne drugs, antiaging drugs, wrinkle defense drugs, bactericides, analgesics, antitussives, antipruritic drugs, local anesthetics, antialopecia agents, hair growth-promoting agents, hair growth-inhibiting agents, anti-dandruff agents, antihistaminic agents, keratolytic agents, anti-inflammatory drugs, refreshing drinks, therapeutic drugs, anti-infective drugs, preventive drugs for inflammation, antiemetics, anticholinergic drugs, vasoconstrictors, vasodilators, trauma healing aids, peptides, polypeptides, proteins, deodorants, antiperspirants, skin softeners, skin moisturizer solutions, softening agents, hair conditioners, hair softeners, hair moisturizers, tanning agents, skin-whitening agents, antifungal agents, depilatories, analgesic drugs for external use, drugs for counterirritation, drugs for hemorrhoids, insecticides, therapeutic drugs for poison ivy rash, therapeutic drugs for poison sumac rash, therapeutic drugs for burns, anti-diaper rash drugs, drugs for heat rash, skin lotions, vitamins, amino acids, amino acid derivatives, herb extracts, retinoid, flavonoid, sense markers, skin conditioners, hair lighteners, chelating agents, cellular turnover enhancers, coloring agents, sunscreens, anesthetics, immunostimulators, revitalizers, water absorbers, sebum absorbers, and mixtures thereof.

The active ingredient used in the present embodiment can also contain, for example, a plant preparation such as extracts or tinctures, for the treatment of local skin disease. Examples of the extracts or tinctures include oak bark extracts, walnut extracts, arnica tinctures, witch hazel extracts, *Plantago lanceolata* extracts, pansy extracts, thyme or sage extracts; St. John's wort tinctures, golden glow extracts, chamomile flower extracts, or calendula tinctures; and, for example, birch leaf extracts, nettle extracts, coltsfoot extracts, comfrey tinctures, horsetail extracts, aloe extracts, *Aesculus turbinata* and *Ruscus aculeatus* extracts, and arnica, calendula, and chili pepper extracts, for a care for heavily tired skin or damaged skin.

The amino acids as the active ingredient that may be used in the present embodiment include not only salts, esters, or acyl derivatives thereof but also amino acids obtained from the hydrolysis of various proteins. Examples of such amino acid drugs include: amphoteric amino acids such as alkylamidoalkylamines, stearyl acetyl glutamate, caproyloyl silk amino acids, and capryloyl collagen amino acids; capryloyl keratin amino acids; capryloyl pea amino acids; cocodimonium hydroxypropyl silk amino acids; corn gluten amino acids; cysteine; glutamic acid; glycine; hair keratin amino acids; hair amino acids such as aspartic acid, threonine, serine, glutamic acid, proline, glycine, alanine, half-cystine, valine, methionine, isoleucine, leucine, tyrosine, phenylalanine, cysteic acid, lysine, histidine, arginine, cysteine, tryptophan, and citrulline; lysine; silk amino acids; wheat amino acids; and mixtures thereof.

The peptides, the polypeptides, and the proteins as the active ingredient that may be used in the present embodiment include, for example, polymers having a long chain whose number of carbon atoms is at least approximately 10, and a high molecular weight of, for example, at least 1000, and they are formed by the self-condensation of amino acids. Examples of such proteins include: collagen; deoxyribonuclease; iodized corn protein; keratin; milk protein; protease; serum protein; silk; sweet almond protein; wheat germ protein; wheat protein; alpha and beta helix of wheat protein or keratin protein; and hair proteins such as intermediate filament protein, high-sulfur content protein, ultrahigh-sulfur content protein, intermediate filament-associated protein, high-tyrosine protein, high-glycine/tyrosine protein, trichohyalin, and mixtures thereof.

Examples of the antiwrinkle ingredients that may be used in the present embodiment include hyaluronic acid, sodium hyaluronate, retinol (vitamin A), silybin peptides (HTC collagen, palmitoyl penta, peptide 3, and Argireline), amino acids, hydroxyproline, tocopheryl retinoate, ursolic acid, vitamin C derivatives, coenzyme Q10, astaxanthin, fullerene, polyphenols, alpha lipoic acid, soybean extracts, pullulan, active isoflavone, sugars, polysaccharides, glycerin, and glycerin derivatives. However, the antiwrinkle ingredients are not limited to these, and mixing is also possible.

Examples of the vitamins used in the present embodiment include: vitamin B complex; vitamins A (e.g., vitamin A palmitate), C, D, E, and K and theirs derivatives, including thiamine, nicotinic acid, biotin, pantothenic acid, choline, riboflavin, vitamin B6, vitamin B12, pyridoxine, inositol, and carnitine; and provitamins such as panthenol (provitamin B5) and panthenol triacetate; and mixtures thereof.

Examples of the antimicrobial agents that may be used in the present embodiment include bacitracin, erythromycin, neomycin, tetracycline, chlortetracycline, benzethonium chloride, phenol, and mixtures thereof.

Examples of the skin softeners and the skin moisturizers that may be used in the present embodiment include mineral oils, lanolin, vegetable oils, isostearyl isostearate, glyceryl laurate, methyl gluceth-10, methyl gluceth-20, chitosan, and mixtures thereof.

Examples of the hair conditioners that may be used in the present embodiment include not only lipophilic compounds such as cetyl alcohol, stearyl alcohol, hydrogenated polydecene, and mixtures thereof, but also quaternary compounds such as behenamidopropyl PG-dimonium chloride, tricetyl ammonium chloride, dihydrogenated tallowamidoethyl hydroxyethylmonium methosulfate, and mixtures thereof.

Examples of the sunscreens that may be used in the present embodiment include butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, methoxycinnamic acid diethanolamine, glycerin aminobenzoate, titanium dioxide, zinc oxide, oxybenzone, Padimate O, red petrolatum, and mixtures thereof. The tanning agent that may be used in the present embodiment is dihydroxyacetone.

Examples of the skin-whitening agents that may be used in the present embodiment include hydroquinone and its derivatives, catechol and its derivatives, ascorbic acid and its derivatives, ellagic acid and its derivatives, kojic acid and its derivatives, tranexamic acid and its derivatives, resorcinol derivatives, placenta extracts, arbutin, oil-soluble licorice extracts, and mixtures thereof.

Examples of the anti-inflammatory analgesics that may be used in the present embodiment include acetaminophen, methyl salicylate, monoglycol salicylate, aspirin, mefenamic acid, flufenamic acid, indomethacin, diclofenac, alclofenac, diclofenac sodium, ibuprofen, ketoprofen, naproxen, pranoprofen, fenoprofen, sulindac, fenclofenac, clidanac, flurbiprofen, fentiazac, bufexamac, piroxicam, phenylbutazone, oxyphenbutazone, clofezone, pentazocine, mepirizole, and tiaramide hydrochloride. Examples of the steroidal anti-inflammatory analgesics that may be used together with the patch of the present embodiment include hydrocortisone, prednisolone, dexamethasone, triamcinolone acetonide, fluocinolone acetonide, hydrocortisone acetate, prednisolone acetate, methylprednisolone, dexamethasone acetate, betamethasone, betamethasone valerate, flumethasone, fluorometholone, and beclomethasone dipropionate.

Examples of the antihistaminic drugs that may be used in the present embodiment include diphenhydramine hydrochloride, diphenhydramine salicylate, diphenhydramine, chlorpheniramine hydrochloride, chlorpheniramine maleate, isothipendyl hydrochloride, tripelennamine hydrochloride, promethazine hydrochloride, and methdilazine hydrochloride. Examples of the local anesthetics that may be used together with the patch of the present embodiment include dibucaine hydrochloride, dibucaine, lidocaine hydrochloride, lidocaine, benzocaine, p-buthylaminobenzoic acid 2-(diethylamino) ethyl ester hydrochloride, procaine hydrochloride, tetracaine, tetracaine hydrochloride, chloroprocaine hydrochloride, oxyprocaine hydrochloride, mepivacaine, cocaine hydrochloride, piperocaine hydrochloride, dyclonine, and dyclonine hydrochloride.

Examples of the bactericides and the disinfectants that may be used in the present embodiment include thimerosal, phenol, thymol, benzalkonium chloride, benzethonium chloride, chlorhexidine, povidone iodine, cetylpyridinium chloride, eugenol, and trimethylammonium bromide. Examples of the vasoconstrictors that may be used together with the patch of the present embodiment include naphazoline nitrate, tetrahydrozoline hydrochloride, oxymetazoline hydrochloride, phenylephrine hydrochloride, and tramazoline hydrochloride. Examples of the hemostats that may be used together with the patch of the present embodiment include thrombin, phytonadione, protamine sulfate, aminocaproic acid, tranexamic acid, carbazochrome, carbazochrome sodium sulfonate, rutin, and hesperidin.

Examples of the chemotherapeutic drugs that may be used in the present embodiment include sulfamine, sulfathiazole, sulfadiazine, homosulfamine, sulfisoxazole, sulfisomidine, sulfamethizole, and nitrofurazone. Examples of the antibiotics that may be used together with the patch of the present embodiment include penicillin, methicillin, oxacilline, cephalothin, cephalodine, erythromycin, lincomycin, tetracycline, chlorotetracycline, oxytetracycline, methacycline, chloramphenicol, kanamycin, streptomycin, gentamycin, bacitracin, and cycloserine.

Examples of the antiviral drugs that may be used in the present embodiment include protease inhibitors, thymidine kinase inhibitors, sugar or glycoprotein synthesis inhibitors, constituent protein synthesis inhibitors, adherence and adsorption inhibitors, and nucleoside analogs such as acyclovir, penciclovir, valaciclovir, and ganciclovir.

Examples of the hair growth stimulants or hair restorers that may be used in the present embodiment include minoxidil, carpronium chloride, pentadecanoic acid glyceride, tocopherol acetate, piroctone olamine, glycyrrhizic acid, isopropylmethylphenol, hinokitiol, *Swertia japonica* extracts, ceramide and precursors, nicotinic acid amide, and chili pepper tinctures.

Examples of the beauty active ingredients that may be used in the present embodiment include D-alpha tocopherol, DL-alpha tocopherol, D-alpha-tocopheryl acetate, DL-alpha-tocopheryl acetate, ascorbyl palmitate, vitamin F and vitamin F glyceride, vitamin D, vitamin D2, vitamin D3, retinol, retinol ester, retinyl palmitate, retinyl propionate, beta carotene, coenzyme Q10, D-panthenol, farnesol, farnesyl acetate; jojoba oil and black currant oil abundantly contained in essential fatty acid; 5-n-octanoyl salicylic acid and its esters, salicylic acid and its esters; alkyl esters of alpha hydroxy acids such as citric acid, lactic acid, and glycolic acid; asiatic acid, madecassic acid, asiaticoside, total extracts of *Centella asiatica,* beta glycyrrhetinic acid, alpha bisabolol, ceramide such as 2-oleoylamino-1,3-octadecane; phytantriol, phytoplankton-derived phospholipid abundantly contained in poly-unsaturated essential fatty acid, ethoxyquin; rosemary extracts, balm extracts, quercetin, dry microalgal extracts, anti-inflammatory drugs such as steroidal anti-inflammatory drugs, and biochemical stimulants such as hormones or fats and/or compounds attributed to the synthesis of proteins.

The vitamin C used in the present embodiment promotes collagen (connective tissue) synthesis, lipid (fat) and carbohydrate metabolism, and neurotransmitter synthesis. The vitamin C is also essential for the optimum maintenance of the immune system. The vitamin C is harmful to a wide range of cancer cells, particularly, melanoma. Tyrosine oxidase, which catalyzes the aerobic activity of tyrosine that is converted to melanin and other pigments, is also prevented in the presence of vitamin C from being activated. The vitamin C has been found to be effective for catalyzing immune response to infections with many viruses and bacteria. In addition to many applications described above, the vitamin C is essential for collagen synthesis and trauma treatment. The patch to which the present embodiment is applied may contain a combination of vitamin C, vitamin E, and other ingredients such as humectants, collagen synthesis promoters, and facial scrubs.

The skin conditioner ingredients in the present embodiment include mineral oils, Vaseline, vegetable oils (e.g., soybean oil or maleated soybean oil), dimethicone, dimethicone copolyol, cationic monomers and polymers (e.g., guar hydroxypropyltrimonium chloride and distearyl dimethyl ammonium chloride), and mixtures thereof. Examples of the humectants include polyols such as sorbitol, glycerin, propylene glycol, ethylene glycol, polyethylene glycol, polypropylene glycol, 1,3- butanediol, hexylene glycol, isoprene glycol, xylitol, fructose, and mixtures thereof.

These active ingredients may be used alone or may be used in combination of two or more types, and as a matter of course, active ingredients in any form of inorganic salts or organic salts are also included as long as they are pharmaceutically acceptable salts. Moreover, although the active ingredient is basically incorporated in the coating carrier, the active ingredient can also be supplied later via the through-holes 4 formed in the base 2 without being incorporated in the coating carrier. Alternatively, the active ingredient is directly applied to the skin, and then, the array 1 with microprotrusions can also be placed against the same site on the skin. In this case, it becomes possible to promote the permeation of the active ingredient into the skin by virtue of the effect of stretching the skin and the effect of ODT (occlusive dressing therapy) on the skin.

An auxiliary instrument for fixing the array 1 may be used in administration using the array 1. However, direct administration by hand pressing is more preferable than, for example, an array that generates high collision energy as described in JP 2004-510535. When the array 1 comes in contact with the skin, administration is performed with a force of 1.0 to 10 kg or administration is performed with a force of 1.0 to 7 kg or with a force of 1.0 to 4 kg. Moreover, the administration time with this force is not so long and is on the order of a few seconds to 120 minutes at the longest. The administration time may be within 60 minutes or may be within 15 minutes. In some cases, instantaneous administration for less than 1 second is also possible. However, it is also possible to continue to administer the active ingredient by then fixing the array to the skin.

## Examples

Hereinafter, Examples of a device with microprotrusions will be described specifically, and however, the constitution of the device with microprotrusions is not limited to Examples below.

(Example 1) Primary rabbit skin irritation test

<Operational procedure>

Three types of arrays with microprotrusions made of polylactic acid in which the lengths of the protrusions differed (h: 70 $\mu$m, 110 $\mu$m, and 150 $\mu$m), and an array consisting only of a base were used. With regard to three types of arrays with microprotrusions, all the shapes of the protrusions were quadrangular pyramids, and all the densities of the protrusions were 841 protrusions/cm$^2$. A test substance was pressed for 5 seconds with a force of 3 kg against the skin in the shaved dorsal portion of each 18-week-old female Japanese white rabbit (Kbl:JW) and then applied for 2 hours (with a covering member) (the number of individuals n = 6). Then, the test substance was peeled off after 2 hours from administration, and erythema/eschar and edema formation were grossly observed on 0.5, 2, 24, 48, and 72 hours after peeling and graded on the basis of the scoring criteria of Draize et al. (Table 1).

The assessment of primary skin irritation was performed by calculating a primary irritation index (P.I.I.) and using the scoring criteria of Draize (Table 2) shown below. The primary irritation index was calculated by respectively determining the average scores of each individual as to erythema and edema formation after 0.5 hours and 24 hours after test substance (MN) peeling, further determining the total sum of the average scores of each group, and dividing by the number of individuals.

As shown in Table 3, the primary irritation index (P.I.I) of the skin was 0.0 for all of three types of arrays with the respective microprotrusions (70 $\mu$m, 110 $\mu$m, and 150 $\mu$m) and the array with only a base.

[Table 1]

| Skin reaction scoring criteria (Draize) | |
| --- | --- |
| Erythema and eschar formation | Score |
| No erythema | 0 |
| Very slight erythema (barely perceptible) | 1 |
| Well-defined erythema | 2 |
| Moderate to severe erythema | 3 |
| Severe erythema (beet redness) to slight eschar formation (injuries in depth) | 4 |
| Edema formation | Score |
| No edema | 0 |
| Very slight edema (barely perceptible) | 1 |
| Slight edema (perceptible edges of area well defined by definite raising) | 2 |
| Moderate edema (raising approximately 1 mm) | 3 |
| Severe edema (raising approximately 1 mm and extending beyond the area of exposure) | 4 |

[Table 2]

| Scoring criteria of primary skin irritation (Draize) | |
| --- | --- |
| P.I.I. $\leq$ 2 | Mild irritant |
| 2 < P.I.I. $\leq$ 5 | Moderate irritant |
| 5 < P.I.I. $\leq$ 8 | Severe irritant |

[Table 3]

| | N | 30 minutes after MN removal | | 24 hours after MN removal | |
| --- | --- | --- | --- | --- | --- |
| | | Erythema | Edema | Erythema | Edema |
| Base | 6 | 0.0 | 0.0 | 0.0 | 0.0 |
| 70 $\mu$m | 6 | 0.0 | 0.0 | 0.0 | 0.0 |
| 110 $\mu$m | 6 | 0.0 | 0.0 | 0.0 | 0.0 |
| 150 $\mu$m | 6 | 0.0 | 0.0 | 0.0 | 0.0 |

(Example 2) Rabbit skin occult blood test

<Operational procedure>

An array with microprotrusions made of polylactic acid in which the length of the protrusions was 150 $\mu$m was used. In this array, the shape of the protrusions was a quadrangular pyramid, and the density of the protrusions was 841 protrusions/cm$^2$. Similarly to Example 1 above, the array was pressed against the rabbit skin and applied for 2 hours, and then, occult blood reaction was assayed (the number of individuals n = 6). Judgment criteria for occult blood were judged through color reaction when 20 $\mu$L of saline was added dropwise to the application site immediately after removing the array (MN) and test paper was placed thereagainst. Pretest II (urine occult blood test paper, manufactured by Wako Pure Chemical Industries, Ltd.) was used as a test paper for assay. The skin was abraded using an 18-G needle as a positive control and tested similarly.

The occult blood reaction of the protrusion-applied group was observed in none of the rabbits, and the occult blood reaction was negative. On the other hand, with regard to the abraded group, evident coloring (blue) was observed with the test paper, though bleeding was hardly observed grossly. Since the occult blood reaction was not observed as to the microprotrusions of 150 $\mu$m in height, it was expected that the occult blood reaction was not observed as to the

microprotrusions of 150 μm or smaller in height used in the irritation test.

[Table 4]

| | Animal No. | Immediately after MN removal |
| --- | --- | --- |
| | | Occult blood reaction |
| 150 μm | 1 | None |
| | 2 | None |
| | 3 | None |
| | 4 | None |
| | 5 | None |
| | 6 | None |

(Example 3) Rabbit transepidermal water loss (TEWL) assay

<Operational procedure>

Three types of arrays with microprotrusions made of polylactic acid in which the lengths of the protrusions differed (h: 70 μm, 110 μm, and 150 μm), and an array consisting only of a base were used. With regard to three types of arrays with microprotrusions, all the shapes of the protrusions were quadrangular pyramids, and all the densities of the protrusions were 841 protrusions/cm$^2$. Similarly to Example 1 above, each array was applied by pressing against the rabbit skin, and water loss was assayed around 2 hours after application. Transepidermal water loss (TEWL) is an index for assaying the barrier function of the skin, and it has been shown that water loss from a damaged site increases when the barrier structure of the stratum corneum gets damaged.

The assay of transepidermal water loss was conducted before application of the array and after a few minutes from peeling and performed using VapoMeter (manufactured by Dlefin). An array with microprotrusions of 500 μm in height with a similar shape was used for a positive control group, and an array consisting only of a base was used for a negative control group.

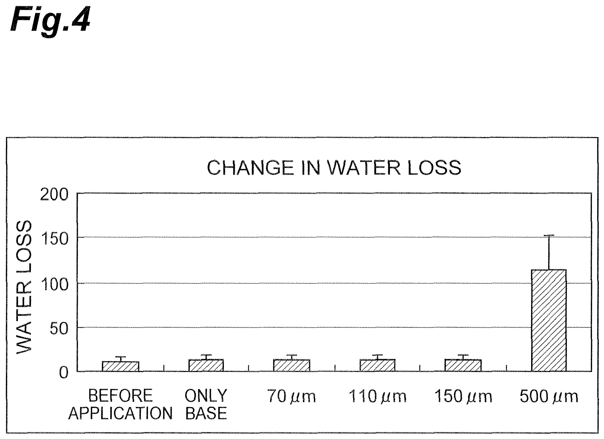

The experimental results are shown in Figure 4. In the case of using the array with microprotrusions of 500 μm in height, an evident rise in water loss was confirmed. On the other hand, although slight increase in water loss was observed for the arrays with microprotrusions of 70 to 150 μm in height, it was shown that the microprotrusions did not have influence on water loss because a similar rising tendency was also observed for the array not provided with microprotrusions.

From these results shown in Figure 4, it was assumed that the arrays with microprotrusions of 70 to 150 μm in height did not give physical damage to the skin under this condition.

(Example 4) Excised human skin impedance assay

<Operational procedure>

Three types of arrays with microprotrusions made of polylactic acid in which the lengths of the protrusions differed (h: 70 μm, 110 μm, and 150 μm), and an array consisting only of a base were used. All the shapes of the protrusions were quadrangular pyramids, and all the densities of the protrusions were 841 protrusions/cm$^2$. Each array was pressed for 5 seconds at a pressure of 3 kg against excised human skin, and the impedance value of the skin was measured before and after pressing. The impedance value of the skin is an index representing the barrier function of the skin as in TEWL, and it has been shown that the impedance value decreases when the skin gets damaged.

The measurement of the skin impedance value was performed by placing a skin fragment (skin thickness: approximately 500 μm, 5 cm × 5 cm) onto a stainless base, placing a round nonwoven fabric of φ14 in diameter impregnated with saline and a silver electrode of φ12 in diameter onto the base application site, and connecting the stainless base and the silver electrode with an LCR meter (3522-50, Hioki E.E. Corp.) (measurement conditions: IV, 10Hz). An array with microprotrusions of 500 μm in height with a similar shape was used for a positive control group, and an array consisting only of a base was used for a negative control group.

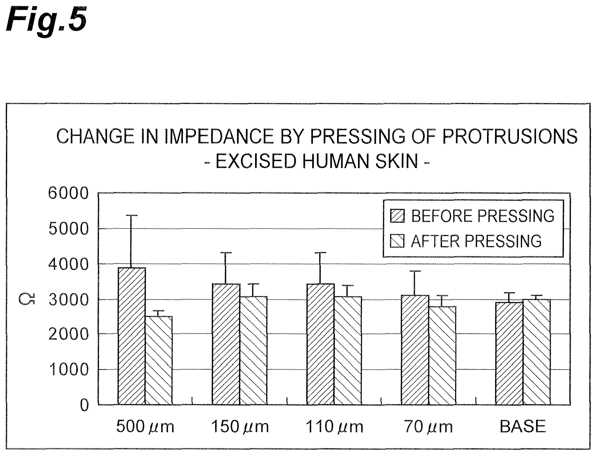

The experimental results are shown in Figure 5. In the case of using the array with microprotrusions of 500 μm in height, the tendency that the impedance value decreased after pressing was observed. On the other hand, in the arrays with microprotrusions of 70 to 150 μm in height, correlation was not observed between the rate of decrease and the height, though a slight decreasing tendency was observed.

(Rate of stretching of skin in Examples 2 to 4)

<Case where skin was stretched completely along side of microprotrusion>

This case will be described using Figures 3(c) and 3(d). In the case where the skin with the length b along the line segment QM in a normal state has been stretched to the length a completely along the line segment PQ, the rate of stretching thereof depends on the apical angle $\alpha$ of the microprotrusion 3, regardless of the length of the microprotrusion 3. As shown in Tables 5 to 7 below, the rates of stretching in the case where the apical angles $\alpha$ were 16, 18, and 20 degrees were 7.19, 6.37, and 5.74, respectively.

[Table 5]

| (Case where apical angle was 16 degrees) | | | |
|---|---|---|---|
| Length | b ($\mu$m) | a ($\mu$m) | Rate of stretching (a/b) |
| 150 $\mu$m | 21.11 | 151.87 | 7.19 |
| 100 $\mu$m | 14.07 | 101.22 | 7.19 |
| 50 $\mu$m | 7.04 | 50.65 | 7.19 |

[Table 6]

| (Case where apical angle was 18 degrees) | | | |
|---|---|---|---|
| Length | b ($\mu$m) | a ($\mu$m) | Rate of stretching (a/b) |
| 150 $\mu$m | 23.9 | 152.23 | 6.37 |
| 100 $\mu$m | 15.9 | 101.27 | 6.37 |
| 50 $\mu$m | 7.95 | 50.64 | 6.37 |

[Table 7]

| (Case where apical angle was 20 degrees) | | | |
|---|---|---|---|
| Length | b ($\mu$m) | a ($\mu$m) | Rate of stretching (a/b) |
| 150 $\mu$m | 26.56 | 152.33 | 5.74 |
| 100 $\mu$m | 17.71 | 101.56 | 5.74 |
| 50 $\mu$m | 8.85 | 50.78 | 5.74 |

<Case where skin was stretched incompletely along side of microprotrusion>

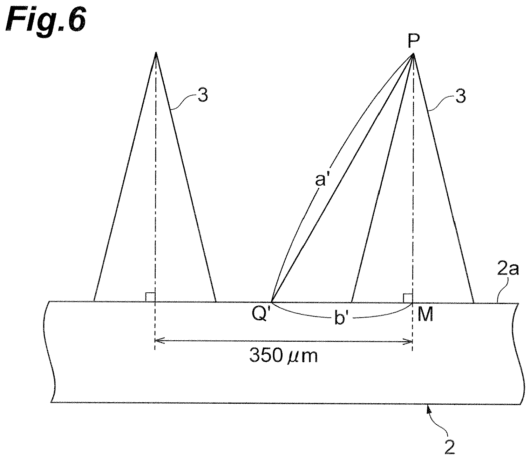

This case will be described using Figure 6. Figure 6 schematically shows the situation in which a portion of the skin was contacted with the base surface 2a between adjacent microprotrusions 3 by the pressing of the array with microprotrusions, so that the skin was not stretched along the side of the microprotrusion 3. If the skin is contacted with the base surface 2a at a midpoint Q' between the microprotrusions 3, the skin with a length b' along a line segment Q'M in a normal state shall be stretched to a length a' along a line segment PQ'. The rate a'/b' of stretching of the skin becomes a value shown in Table 8 below according to the height of the microprotrusions 3 provided that the microprotrusions 3 are arranged with equal spacing of 350 $\mu$m and the apical angle of the microprotrusions 3 is 20 degrees.

[Table 8]

| (Case where apical angle was 20 degrees) | | | |
|---|---|---|---|
| Length | b' ($\mu$m) | a' ($\mu$m) | Rate of stretching (a'/b') |
| 500 $\mu$m | 530.0 | 175 | 3.03 |
| 150 $\mu$m | 230.5 | 175 | 1.32 |
| 100 $\mu$m | 201.6 | 175 | 1.15 |
| 50 $\mu$m | 182.0 | 175 | 1.04 |

**Industrial Applicability**

In an aspect of the present invention, since an active ingredient intended for beauty can be administered to the skin without pain and with reliability, with damage to the stratum corneum of the skin prevented, the convenience of the array with microprotrusions is drastically enhanced, and thus, there is industrial applicability.

**Reference Signs List**

1 ... Array with microprotrusions, 2 ... Base, 3 ... Microprotrusion, 4 ... Through-hole, 5 ... Coating.

**Claims**

1. An array with microprotrusions (1), comprising:

   a base (2); and
   tapered microprotrusions (3) each disposed on the base (2) and tapering down toward the tip from the bottom connected to the base (2), wherein
   denoting the distance from the tip to the bottom on an arbitrary side of each of the microprotrusions (3) as a and denoting the length of a second line segment obtained by projecting a first line segment representing the distance onto the base (2) as b, the ratio a/b satisfies $1.0 < (a/b) \leq 7.5$, the height of the microprotrusions is 50 to 300 $\mu$m, and the tip is flat and the area of the tip is 20 to 600 $\mu$m$^2$.

2. The array with microprotrusions (1) according to claim 1, wherein the microprotrusions (3) are made of polylactic acid.

3. The array with microprotrusions (1) according to claim 1 or 2, wherein the density of the microprotrusions (3) is 100 to 10000 protrusions/cm$^2$.

4. The array with microprotrusions (1) according to any one of claims 1 to 3, wherein an administration using the array with microprotrusions (1) is a direct administration by hand pressing.

**Patentansprüche**

1. Array mit Mikro-Vorsprüngen (1), das umfasst:

   einen Träger (2); sowie
   sich verjüngende Mikro-Vorsprünge (3), die jeweils auf dem Träger (2) angeordnet sind und sich von dem oberen Ende zu dem mit dem Träger (2) verbundenen unteren Ende verjüngen, wobei
   wenn der Abstand von dem oberen Ende zu dem unteren Ende an einer beliebigen Seite jedes der Mikro-Vorsprünge (3) mit "a" bezeichnet wird und die Länge einer zweiten Strecke, die ermittelt wird, indem eine erste Strecke, die den Abstand repräsentiert, auf den Träger (2) projiziert wird, mit "b" bezeichnet wird, für das Verhältnis a/b $1,0 < (a/b) \leq 7,5$ gilt, die Höhe der Mikro-Vorsprünge 50 bis 300 $\mu$m beträgt und das obere Ende flach ist und die Fläche des oberen Endes 20 bis 600 $\mu$m$^2$ beträgt.

2. Array mit Mikro-Vorsprüngen (1) nach Anspruch 1, wobei die Mikro-Vorsprünge (3) aus Polymilchsäure bestehen.

**3.** Array mit Mikro-Vorsprüngen (1) nach Anspruch 1 oder 2, wobei die Dichte der Mikro-Vorsprünge (3) 100 bis 10.000 Vorsprünge/cm$^2$ beträgt.

**4.** Array mit Mikro-Vorsprüngen (1) nach einem der Ansprüche 1 bis 3, wobei eine Verabreichung unter Verwendung des Array mit Mikro-Vorsprüngen (1) eine direkte Verabreichung durch Drücken mit der Hand ist.

**Revendications**

**1.** Réseau à microprotubérances (1), comprenant:

un socle (2); et
des microprotubérances effilées (3) disposées chacune sur le socle (2) et se rétrécissant vers la pointe en partant du bas et reliées au socle (2), dans lequel
on désigne par a la distance entre l'extrémité et le bas d'un côté arbitraire de chacune des microprotubérances (3) et on désigne par b la longueur d'un second segment de droite obtenu en projetant un premier segment de droite représentant la distance sur la base (2), le rapport a/b satisfait à 1,0 < (a/b) ≤ 7,5, et la hauteur des microprotubérances est comprise entre 50 et 300 $\mu$m, la pointe est plate et la surface de la pointe est comprise entre 20 et 600 $\mu$m$^2$.

**2.** Réseau à microprotubérances (1) selon la revendication 1, dans lequel les microprotubérances (3) sont en acide polylactique.

**3.** Réseau à microprotubérances (1) selon les revendications 1 ou 2, dans lequel la densité des microprotubérances (3) est 100 à 10000 protubérances/cm$^2$.

**4.** Réseau à microprotubérances (1) selon l'une quelconque des revendications 1 à 3, dans lequel une administration utilisant le réseau à microprotubérances (1) est une administration directe par pressage manuel.

# Fig.1

**Fig.2**

# *Fig.3*

(a)

3

B       B

(b)

P

3

α

a

Q   b   M     R

(c)

3

D       D

(d)

P

3

α

a

Q   b M     R

# Fig.4

CHANGE IN WATER LOSS

# *Fig.5*

CHANGE IN IMPEDANCE BY PRESSING OF PROTRUSIONS
- EXCISED HUMAN SKIN -

## Fig.6

**EP 2 578 265 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001506904 A
- JP 2004504120 A
- JP 2007089792 A
- JP 2007130417 A
- JP 2004510535 A